(19) Europäisches Patentamt
European Patent Office
Office européen des brevets

(11) Veröffentlichungsnummer: **0 164 663**
**B1**

# (12) EUROPÄISCHE PATENTSCHRIFT

(45) Veröffentlichungstag der Patentschrift: **10.01.90**

(51) Int. Cl.⁴: **C 08 L 33/12**, C 08 K 5/17, C 08 F 20/14, C 08 K 5/34, A 61 N 5/06

(21) Anmeldenummer: **85106721.5**

(22) Anmeldetag: **31.05.85**

(54) **Kunststoff mit hoher UV-Durchlässigkeit und Verfahren zu seiner Herstellung.**

(30) Priorität: **13.06.84 DE 3421859**

(43) Veröffentlichungstag der Anmeldung: **18.12.85 Patentblatt 85/51**

(45) Bekanntmachung des Hinweises auf die Patenterteilung: **10.01.90 Patentblatt 90/2**

(84) Benannte Vertragsstaaten: **DE FR GB NL SE**

(56) Entgegenhaltungen: **EP-A- 0 016 870**

(73) Patentinhaber: **Röhm GmbH, Kirschenallee Postfach 4242, D-6100 Darmstadt 1 (DE)**

(72) Erfinder: **Hosch, Ludwig, Fritz-Kredel-Strasse 6, D-6120 Michelstadt (DE)**
Erfinder: **Schneider, Albert, Magdeburger Strasse 24, D-6107 Reinheim 1 (DE)**
Erfinder: **Wunderlich, Winfried, Dr., Spessartring 11, D-6101 Rossdorf 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft einen Kunststoff auf Basis von Methylmethacrylat mit dauerhaft hoher Strahlungs-Durchlässigkeit im UV-Bereich sowie ein Verfahren zu seiner Herstellung. Die Erfindung betrifft weiterhin den technischen Einsatz des Kunststoffes in einem Solarium oder als UVdurchlässige Abdeckung.

Aus der EP-B 16870 sind bereits Kunststoffe auf Basis von Methylmethacrylat bekannt, die eine hohe Durchlässigkeit für UV-A-Strahlung (300–400 nm) und für UV-B-Strahlung (260–320 nm) haben. Die hohe UV-Durchlässigkeit bleibt auch bei langer Strahlungseinwirkung erhalten, wenn der Kunststoff eine geringe Menge eines sterisch gehinderten Amins der Formel

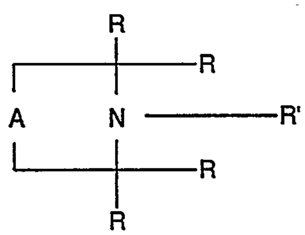

worin R gleiche oder verschiedene Alkylreste, R' ein Wasserstoffatom oder ein Alkylrest und A eine gegebenenfalls substituierte 2- oder 3-gliedrige Alkylenkette darstellen, enthält. Während die UV-Durchlässigkeit bei Kunststoffen auf Methylmethacrylatbasis innerhalb einer Bestrahlungszeit von 1000 Stunden von etwa 80 auf 12% absinkt, haben die Kunststoffe mit einem Gehalt des erwähnten sterisch gehinderten Amins nach einer gleichlangen Bestrahlungszeit noch eine Durchlässigkeit von 50%. Wenn am Aufbau des Kunststoffes neben Methylmethacrylat noch 12% eines Acrylesters, wie Methylacrylat beteiligt ist, so ist bei den Kunststoffen, die die genannten sterisch gehinderten Amine enthalten, in einer Bestrahlungszeit von 1000 Stunden praktisch keine Verminderung der UV-Durchlässigkeit mehr festzustellen.

Es wurde nun gefunden, dass die UV-Durchlässigkeit von Kunststoffen auf Methylmethacrylatbasis mit einem Gehalt von 0,01 bis 1 Gew.-% der erwähnten sterisch gehinderten Amine bei lang andauernder UV-Bestrahlung sogar noch ansteigt und auch bei einer Bestrahlungsdauer bis zu 4000 Stunden nicht wieder absinkt, wenn der Kunststoff 1 bis 10 Gew.-% eines aliphatischen, mit Polymethylmethacrylat verträglichen, über 120 °C siedenden Weichmachers enthält. Die vorteilhaften Eigenschaften gehen aus der nachfolgenden Tabelle 1 hervor:

Tabelle 1

UV-Durchlässigkeit von Polymethylmethacrylat mit einem Gehalt an 0,1 Gew.-% Di(2,2,6,6-Tetramethyl-pyridyl-)sebazat und 3 bis 10 Gew.-% aliphatischen Weichmachern

| Beispiel | Weichmacher | Gew.-% | Vicat-Erweichungstemperatur [°C] | Bestrahlungsdauer [Stunden] | % UV-Durchlässigkeit (τ 300 nm) UVA vor Bestrahlung | UVA nach Bestrahlung | UV-B vor Bestrahlung | UV-B nach Bestrahlung |
|---|---|---|---|---|---|---|---|---|
| 1 | Diäthyladipat | 5 | 96 | 3000 | 77 | 85 | 77 | 86 |
| 2 | Diäthyladipat | 10 | 76 | 4000 | 76 | 83 | 76 | 84 |
| 3 | Diäthlenglykolmonomethyläther | 3 | 104 | 1000 | 77 | 79 | 77 | 80 |
| 4 | Diäthlenglykolmonomethyläther | 5 | 95 | 3000 | 83 | 86 | 83 | 87 |
| 5 | Diäthlenglykolmonomethyläther | 10 | 70 | 4000 | 82 | 86 | 82 | 85 |
| 6 | Glycerintriacetat | 5 | 102 | 3000 | 81 | 82 | 81 | 82 |
| 7 | Glycerintriacetat | 10 | 85 | 4000 | 78 | 85 | 78 | 85 |
| Vergleichsversuch | Dibutylphthalat | 5 | 103 | 1000 | 32 | 12 | 32 | 3 |

Wie die zum Vergleich angefügte letzte Zeile der Tabelle zeigt, führen aromatische Weichmacher, wie Dibutylphthalat, zu einer niedrigen, bei Bestrahlung weiter absinkenden UV-Durchlässigkeit.

Wenn es auch bekannt war, dass sich der Zusatz eines äusseren Weichmachers auf Polymerisate des Methylmethacrylats ähnlich auswirkt wie die Mitverwendung von Acrylestern bei der Herstellung des Polymerisats, nämlich durch eine Senkung der Erweichungstemperatur, so war es doch völlig unvorhersehbar, dass mit der Senkung der Erweichungstemperatur eine Verbesserung der UV-Durchlässigkeit im Langzeitverhalten einhergeht. Insbesondere war es überraschend, dass die Wirkung des Weichmacherzusatzes sogar noch über die Wirkung eines Acrylatzusatzes hinausgeht und einen Anstieg der UV-Durchlässigkeit bewirkt.

Das Methylmethacrylat-Polymere kann vollständig aus diesem Monomeren aufgebaut sein, was bevorzugt ist. Neben Methylmethacrylat können jedoch auch bis zu 10 Gew.-%, bezogen auf das Polymere, andere damit mischbare Comonomere am Aufbau des Polymerisats beteiligt sein. Hierzu gehören andere Ester der Methacrylsäure, insbesondere solche von $C_2$–$C_8$-Alkanolen, Alkylester der Acrylsäure, insbesondere mit 1 bis 8 C-Atomen im Alkylrest, sowie gegebenenfalls vernetzende Comonomere, wie Ethylenglykoldimethacrylat und ähnliche. Weniger geeignet sind Comonomere, die eine Absorption im UV-Bereich haben, vor allem Monomere mit aromatischen Gruppen. Dazu gehören beispielsweise Styrol, Vinyltoluol und Benzylacrylat und -methacrylat.

Bei der Herstellung der neuen Kunststoffe erweist es sich als vorteilhaft, dass auf die unangenehm riechenden Acrylester verzichtet werden kann.

Das Molekulargewicht des Methylmethacrylat-Polymeren kann in dem für Kunststoff auf dieser Basis üblichen Bereich liegen. Vorzugsweise liegt es über 500000, insbesondere im Bereich von etwa 1 bis 2 Millionen. Kunststoff mit einem so hohen Molekulargewicht sind nicht extrudier- oder spritzbar und werden daher unmittelbar in Gestalt von Platten oder Rohren polymerisiert. In der Regel wird die Polymerisation in einer Flachkammer durchgeführt, die aus zwei parallel in einem Abstand von 2–20 mm angeordneten Glasscheiben und einer am Rand umlaufenden Dichtungsschnur gebildet wird. Man erhält den Kunststoff dann in Form einer 2 bis etwa 18 mm dicken Platte, die sich im thermoelastisch erweichten Zustand biegen, recken oder kuppel- bzw. muldenförmig umformen lässt.

Die Vicat-Erweichungstemperatur (nach DIN 53460) von reinem Polymethylmethacrylat liegt bei 118 °C. Durch die Mitverwendung von Comonomeren und durch den erfindungsgemässen Zusatz eines Weichmachers wird die Vicat-Erweichungstemperatur gesenkt und liegt z.B. im Bereich zwischen 75 und 115 °C oder zwischen 70 und 110 °C, vorzugsweise zwischen 95 und 105 °C. Der Restmonomerengehalt sollte möglichst unter 0,5%, vorzugsweise unter 0,2%, bezogen auf das Gewicht des Kunststoffes, liegen.

Die sterisch gehinderten Amine, die in dem neuen Kunststoff enthalten sind, sind die gleichen, die in den Kunststoffen gemäss EP-B 16870 verwendet werden. Sie haben die allgemeine Formel

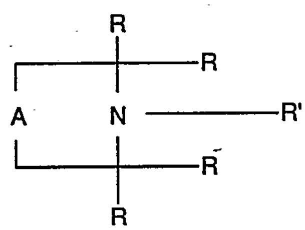

worin R gleiche oder verschiedene Alkylreste, R′ ein Wasserstoffatom oder einen Alkylrest und A eine gegebenenfalls substituierte 2- oder 3-gliedrige Alkylenkette darstellt. Die Reste R sind in der Regel niedere Alkylreste mit höchstens 4-C-Atomen, vorzugsweise Methylgruppen. R′ ist vorzugsweise ein Wasserstoffatom, oder ein niederer Alkylrest, insbesondere Methylrest. R′ kann aber auch ein grösserer aliphatischer Rest sein, mit welchem die Stickstoffatome zweier Aminreste obiger Formel miteinander verknüpft sind. Dieser aliphatische Rest kann beispielsweise eine oder mehrere Estergruppen aufweisen. A ist vorzugsweise eine 3-gliedrige Alkylenkette, so dass das Stickstoffatom einen Piperidylrest, insbesondere einem 2,2,6,6-Tetramethylpiperidylrest angehört. In der Regel trägt das mittlere C-Atom der 3-gliedrigen Alkylenkette einen Substituenten, der gegebenenfalls di- oder polyfunktionell sein kann und zwei oder mehr Gruppen der Formel

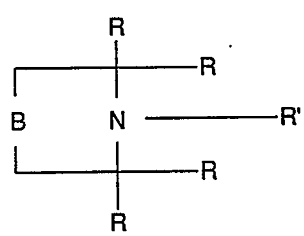

verbinden kann, worin B den vorher mit A bezeichneten Alkylenrest darstellt, wobei jedoch eines der C-Atome eine freie Valenz enthält. Wenn bei der Herstellung des Kunststoffes ein sterisch gehindertes Amin eingesetzt wird, das als Substituenten in der Gruppe A einen ungesättigten, radikalisch polymerisierbaren Rest enthält, beispielsweise einen Acryloxy-, Methacryloxy-, Acrylamido- oder Methacrylamido-Rest, so wird das tertiäre Amin bei der Polymerisation mit den Methylmethacrylat-Polymeren verknüpft. Es entsteht also ein Mischpolymerisat, welches Seitengruppen mit der zuletzt dargestellten Formel enthält. Typische Vertreter der sterisch gehinderten Amine sind:

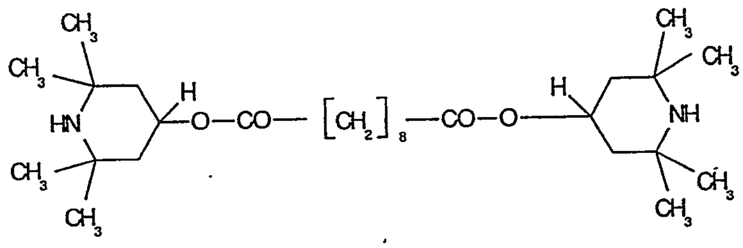

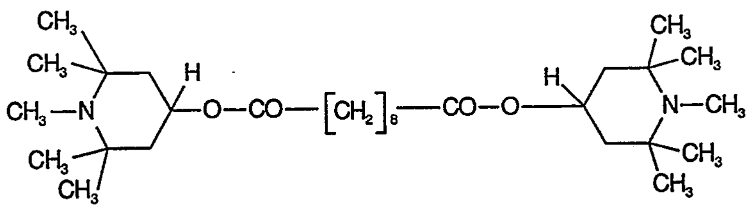

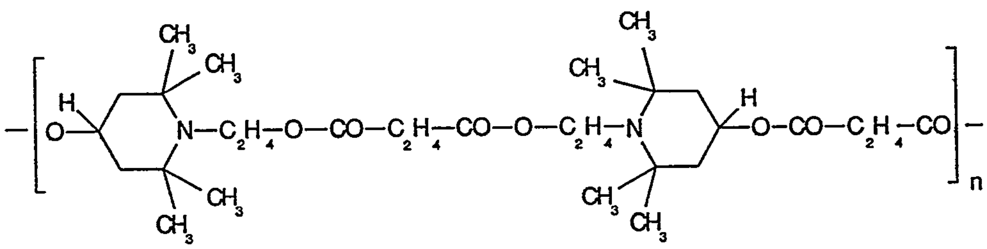

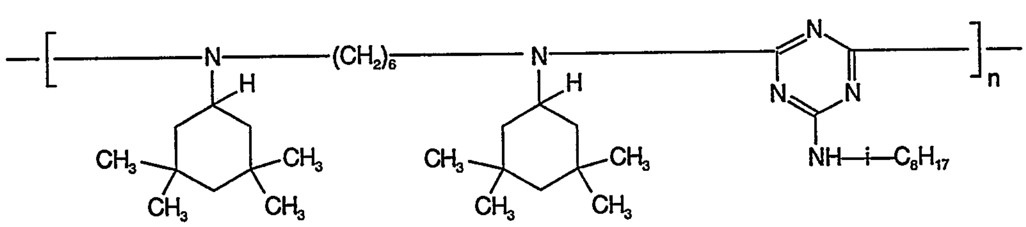

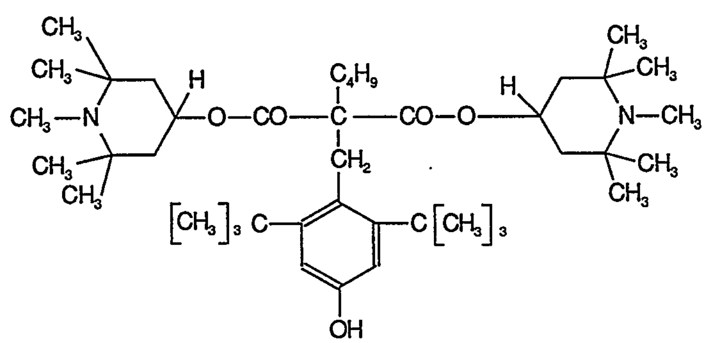

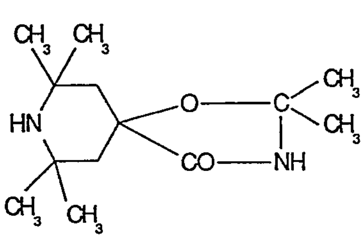

Polymerisierte Einheiten von Verbindungen der Formel

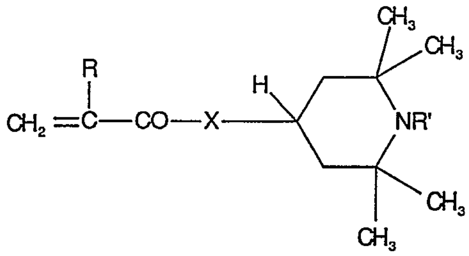

worin R = H oder $CH_3$, R' = H oder $CH_3$ und X = -O- oder -NH- ist, können Bestandteil des Polymerisats sein.

Die dauerhaft hohe und bei längerer Strahlungseinwirkung noch zunehmende Durchlässigkeit für UV-Strahlung beruht auf dem Gehalt des erfindungsgemässen Kunststoffes an einem aliphatischen Weichmacher. Als bevorzugte Weichmacher sind alle aliphatischen Verbindungen anzusehen, die bei einer Zusatzmenge von 1–10 Gew.-% die Vicat-Erweichungstemperatur nach DIN 53460 von Polymethylmethacrylat auf 70 bis 110°C, vorzugsweise 95 bis 105°C senken. Das setzt zunächst voraus, dass sie mit Polymethylmethacrylat verträglich sind. Die Verträglichkeit lässt sich leicht feststellen, wenn man 100 Teile Polymethylmethacrylat und 1 bis 10 Teile des Weichmachers in einem leicht flüchtigen organischen Lösungsmittel, wie Chloroform, löst und aus der Lösung einen dünnen Film herstellt. Wenn nach dem Verdunsten des Lösungsmittels der Film völlig klar ist, ist der Weichmacher mit dem Polymethylmethacrylat verträglich. Ist er dagegen unverträglich, so wird der Film beim Trocknen trüb. Obwohl es in besonderen Fällen erwünscht sein kann, den Weichmacher aus dem Kunststoff während oder nach seiner Verarbeitung wieder zu entfernen, soll er als dauerhafter Bestandteil in dem Kunststoff verbleiben. Weichmacher mit einem Siedepunkt über 120°C entweichen in der Regel nicht von selbst aus dem Kunststoff. Vorzugsweise werden hochsiedende Weichmacher mit einem weit über 120°C liegenden Siedepunkt verwendet.

Zahlreiche in der Kunststofftechnik gebräuchliche organische Weichmacher sind für die vorliegende Erfindung nicht geeignet, nämlich Weichmacher mit aromatischen Gruppen. Dazu gehören vor allen Phthalsäureester. Die erfindungsgemäss verwendeten Weichmacher sind aliphatischer Natur. Ihre Molekühle sind aus Alkyl- bzw. Alkylengruppen aufgebaut und enthalten eine oder mehrere Ester-, Äther- oder Hydroxyfunktionen. Typische Weichmacher dieser Art enthalten 2 bis 4 dieser Funktionen. Beispiele für aliphatische Weichmacher des erfindungsgemäss verwendeten Typs sind Diäthyladipat, Diäthylenglykolmonoäthyläther, Glycerintriacetat oder Diäthylenglykol.

Die Durchlässigkeit für UV-Strahlung sowie ihr Anstieg im Laufe der Bestrahlung nehmen mit steigendem Gehalt des Weichmachers zu. Da jedoch im gleichen Masse die Vicat-Erweichungstemperatur sinkt, muss ein Kompromiss zwischen der erwünschten UV-Durchlässigkeit einerseits und einer noch ausreichenden Vicat-Erweichungstemperatur andererseits eingegangen werden. Wenn der Kunststoff bei seiner Anwendung nicht erhöhten Temperaturen ausgesetzt ist, kann eine spürbare Verminderung der Vicat-Erweichungstemperatur hingenommen werden; in diesem Falle können Weichmacherzusätze von 5 bis 10 Gew.-% angewendet werden. Wenn dagegen die Vicat-Erweichungstemperatur nur geringfügig vermindert werden darf, sollte der Weichmacherzusatz 1 bis 3 Gew.-% nicht übersteigen. In den meisten Fällen ist eine Zusatzmenge von 3 bis 5% am günstigsten, weil in diesem Bereich schon eine dauerhaft hohe UV-Durchlässigkeit und Beständigkeit erreicht wird, während die Erweichungstemperatur noch nicht entscheidend erniedrigt wird.

Die Herstellung der neuen Kunststoffe erfolgt in der Regel durch radikalische Polymerisation eines Gemisches aus

A) wenigstens 90 Gew.-% (bezogen auf die Gewichtssumme von A und B) Methylmethacrylat,

B) 0 bis 10 Gew.-% (bezogen auf die Gewichtssumme von A und B) eines damit copolymerisierbaren Comonomeren, ausgenommen solche, die der nachfolgenden Gruppe C angehören,

C) 0,01 bis 1 Gew.-% (bezogen auf das Gesamtgewicht des Gemisches) eines sterisch gehinderten Amins der in Anspruch 1 angegebenen Formel, darunter

D) einem üblichen radikalischen Initiator oder Initiatorsystem und

E) 1 bis 10 Gew.-% (bezogen auf das Gesamtgewicht des Gemisches) des aliphatischen Weichmachers.

Das Gemisch wird in an sich bekannter Weise auf eine Temperatur gebracht, bei der der radikalische Initiator oder das Initiatorsystem die Polymerisation auslöst. Verwendbar sind alle für die radikalische Polymerisation bekannten Initiatoren vom Peroxid- oder Azo-Typ. Aliphatische Initiatoren werden bevorzugt, da aromatische Initiatoren, wie Dibenzoylperoxid, UV-absorbierende Zerfallsprodukte ergeben. Beispiele geeigneter aliphatischer Initiatoren sind Azo-Bis-Isobutyronitril, Dilauroylperoxyd, tert.-Butyl-Perpivalat, -Peroctoat oder -Perneodecanoat oder Diisopropylperoxidicarbonat. Die Polymerisationstemperatur richtet sich nach der Zerfallstemperatur des Initiators und liegt vorzugsweise im Bereich von 30 bis 80°C, in der Endstufe bis zu 120°C. Die Polymerisation dauert in der Regel 15 bis 20 Stunden. Ein möglichst vollständiger Umsatz ist anzustreben; der Restmonomerengehalt soll möglichst unter 0,5 vorzugsweise unter 0,2% liegen. Wie oben schon erwähnt, wird die Polymerisation vozugsweise in einer aus zwei Glasscheiben und einer Dichtungsschnur gebildeten Flachkammer durchgeführt. In diesem Falle kann ein vorpolymerisiertes Monomerengemisch in die Flachkammer gefüllt und darin polymerisiert werden. Die auf diese Weise hergestellten Kunststoffplatten eignen sich im besonderen Masse

zur Herstellung von Solarien-Liegen. Sie können auch zur Herstellung von Abdeckungen für UV-Strahler, Gewächshaus- und Schwimmhallenverglasungen u. dergl. verwendet werden.

## Beispiele

In Flachkammern, die aus zwei Glasscheiben und einer dazwischen am Rand umlaufenden, 5 mm dicken Dichtungsschnur aufgebaut waren, wurden die in der nachfolgenden Tabelle 2 angegebenen Monomer-Mischungen gefüllt. Diese enthielten neben den dort genannten Bestandteilen jeweils 0,01 Gew.-% Azo-bis-isobutyronitril und 0,1 Gew.-% Di(2,2,6,6-Tetramethylpiperidyl)-sebazat. Beim Erwärmen auf die angegebenen Temperaturen entstanden durch Polymerisation klare, farblose Polymerisatplatten.

| Beispiel | Methylmethacrylat (Gew.teile) | Weichmacher (Gew.teile) | Polymerisations-bedingungen | | VET[1)] °C | Remo[2)] % |
|---|---|---|---|---|---|---|
| 1 | 95 | 5 Diäthyladipat | 20 h<br>+5 h | 40°–45°<br>105° | 96 | |
| 2 | 90 | 10 Diäthyladipat | 15 h<br>+5 h | 40°<br>100° | 76 | 0,16 |
| 3 | 97 | 3 Diäthylenglykol-monomethyläther | 15 h<br>+6 h | 40°<br>100° | 104 | |
| 4 | 95 | 5 Diäthylenglykol-monomethyläther | 20 h<br>+5 h | 40°–45°<br>105° | 95 | |
| 5 | 90 | 10 Diäthylenglykol-monomethyläther | 20 h<br>+5 h | 40°–45°<br>90° | 70 | 0,15 |
| 6 | 95 | 5 Glycerintriacetat | 20 h<br>+5 h | 40°–45°<br>105° | 102 | |
| 7 | 90 | 10 Glycerintriacetat | 15 h<br>5 h | 38°<br>90° | 85 | 0,15 |

1) Vicat-Erweichungstemperatur nach DIN 53 460
2) Restmonomerengehalt in Gew.-% des fertigen Polymerisats

## Patentansprüche

1. Kunststoff, enthaltend ein zu wenigstens 90 Gew.-% aus Methylmethacrylat und zu höchstens 10 Gew.-% aus damit mischpolymerisierbaren Comonomeren aufgebautes Polymerisat mit einem Gehalt von 0,01 bis 1 Gew.-% eines sterisch gehinderten Amins der Formel

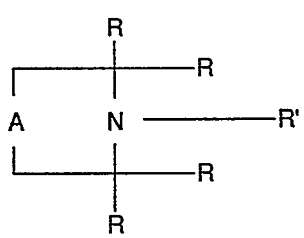

worin R gleich oder verschiedene Alkylreste, R′ ein Wasserstoffatom oder ein aliphatischer Rest und A eine gegebenenfalls substituierte 2- oder 3-gliedrige Alkylenkette darstellen, welches in dem Kunststoff homogen verteilt ist oder über das Glied A mit dem Polymerisat verknüpft ist, dadurch gekennzeichnet, dass der Kunststoff 1 bis 10 Gew.-% eines aliphatischen mit Polymethylmethacrylat verträglichen, über 120°C siedenden Weichmachers enthält.

2. Kunststoff nach Anspruch 1, gekennzeichnet durch ein Molekulargewicht des Methylmethacrylat-Polymerisat über 500000.

3. Kunststoff nach den Ansprüchen 1 oder 2, dadurch gekennzeichnet, dass der aliphatische Weichmacher eine Verbindung mit einer oder mehreren Ester-, Äther- oder Hydroxyfunktionen ist.

4. Kunststoff nach Anspruch 3, dadurch gekennzeichnet, dass der aliphatische Weichmacher eine Verbindung mit zwei bis vier Ester-, Äther- oder Hydroxyfunktionen ist.

5. Kunststoff nach den Ansprüchen 1 bis 4, gekennzeichnet durch einen Restmonomergehalt unter 0,5 Gew.-%.

6. Kunststoff nach den Ansprüchen 1 bis 5, gekennzeichnet durch eine Vicat-Erweichungstemperatur nach DIN 53460 zwischen 75 und 115°C.

7. Kunststoff nach den Ansprüchen 1 bis 6, in Form einer 2 bis 18 mm dicken Platte.

8. Verfahren zur Herstellung eines Kunststoffes gemäss den Ansprüchen 1 bis 6 durch Polymerisation eines Gemisches aus

A) wenigstens 90 Gew.-% (bezogen auf die Gewichtssumme A und B) Methylmethacrylat

B) 0 bis 10 Gew.-% (bezogen auf das Gesamtgewicht des Gemisches) eines damit copolymerisierbaren Comonomeren

C) 0,01 bis 1 Gew.-% (bezogen auf das Gesamtgewicht des Gemisches) eines sterisch gehinderten Amins der in Anspruch 1 angegebenen Formel

D) einem üblichen radikalischen Initiator oder Initiatorsystem, dadurch gekennzeichnet, dass man dem Gemisch 1 bis 10 Gew.-% (bezogen auf das Gesamtgewicht) eines aliphatischen, mit Polymethylmethacrylat verträglichen, über 120°C siedenden Weichmachers zusetzt.

9. Verfahren nach Anspruch 8, dadurch gekennzeichnet, dass die Polymerisation in einer aus zwei parallel in einem Abstand von 2 bis 20 mm angeordneten Glasscheiben und einer am Rand umlaufenden Dichtungsschnur gebildeten Flachkammer durchgeführt wird.

10. Solarium, enthaltend eine Kunststoffplatte gemäss Anspruch 7.

**Claims**

1. Plastics containing a polymer made up of at least 90% by weight of methylmethacrylate and not more than 10% by weight of comonomers which are copolymerisable therewith, said polymer containing 0.01 to 1% by weight of a sterically hindered amine of formula

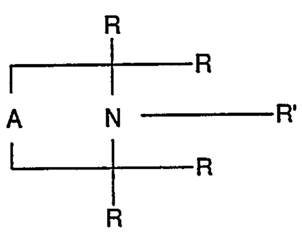

wherein R represents identical or different alkyl groups, R′ represents a hydrogen atom or an aliphatic group and A represents an optionally substituted 2- or 3-membered alkylene chain, said amine being homogeneously distributed in the plastics or linked to the polymer via the member A, characterised in that the plastics contains 1 to 10% by weight of an aliphatic plasticiser which is compatible with polymethyl-methycrylate and has a boiling point above 120°C.

2. Plastics according to claim 1, characterised in that the methylmethacrylate polymer has a molecular weight of above 500,000.

3. Plastics according to claim 1 or 2, characterised in that the aliphatic plasticiser is a compound having one or more ester, ether or hydroxy functions.

4. Plastics according to claim 3, characterised in that the aliphatic plasticiser is a compound having two to four ester, ether or hydroxy functions.

5. Plastics according to claims 1 to 4, characterised in that the residual monomer content is less than 0.5% by weight.

6. Plastics according to claims 1 to 5, characterised in that the Vicat softening temperature according to DIN 53460 is between 75 and 115°C.

7. Plastics according to claims 1 to 6, in the form of a sheet 2 to 18 mm thick.

8. Process for preparing a plastics according to claims 1 to 6 by polymerisation of a mixture of

A) at least 90% by weight (based on the total weigths of A and B) of methylmethacrylate

B)0 to 10% by weight (based on the total weight of the mixture) of a comonomer which is copolymerisable therewith

C) 0.01 to 1% by weight (based on the total weight of the mixture) of a sterically hindered amine of the formula given in claim 1

D) a conventional radical initiator or initiator system, characterised in that 1 to 10% by weight (based on the total weight of an aliphatic plasticiser which is compatible with polymethylmethacrylate and has a boiling point above 120°C is added to the mixture.

9. Process according to claim 8, characterised in that the polymerisation is carried out in a flat chamber formed by two panes of glass arranged parallel and at a spacing of 2 to 20 mm and a sealing cord running around the edge.

10. Solarium containing a sheet of plastics according to claim 7.

**Revendications**

1. Matière plastique contenant un polymère, constitué d'au moins 90% en poids de méthacrylate de méthyle et au plus 10% en poids d'un comonomère copolymérisable avec ce dernier, contenant de 0,01 à 1% en poids d'une amine à empêchement stérique ayant la formule suivante:

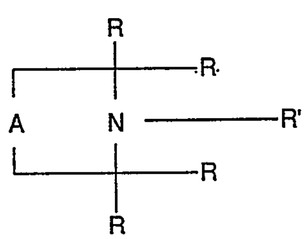

dans laquelle les radicaux R, qui peuvent être identiques ou différents, représentent des radicaux alkyle, R′ est un atome d'hydrogène ou un radical aliphatique, et A est un enchaînement alkylène à 2 ou 3 chaînons, éventuellement substitué, amine qui est répartie d'une manière homogène dans la matière plastique ou est reliée au polymère par l'intermédiaire du chaînon A, caractérisée en ce que la matière plastique contient de 1 à 10% en poids d'un plastifiant aliphatique, dont le point d'ébullition est supérieur à 120°C et qui est compatible avec le poly(méthacrylate de méthyle).

2. Matière plastique selon la revendication 1, caractérisée par une masse moléculaire du polymère de méthacrylate de méthyle supérieur à 500000.

3. Matière plastique selon les revendications 1 ou 2, caractérisée en ce que le plastifiant aliphatique est un composé comportant une ou plusieurs fonctions ester, éther ou hydroxy.

4. Matière plastique selon la revendication 3, caractérisée en ce que le plastifiant aliphatique est un composé comportant de 2 à 4 fonctions ester, éther ou hydroxy.

5. Matière plastique selon les revendications 1 à 4, caractérisée par une teneur en monomère résiduel inférieure à 0,5% en poids.

6. Matière plastique selon les revendications 1 à 5, caractérisée par une température de ramollissement de Vicat, selon DIN 53460, comprise entre 75 et 115°C.

7. Matière plastique selon les revendications 1 à 6, sous la forme d'une plaque d'épaisseur de 2 à 18 mm.

8. Procédé pour la préparation d'une matière plastique selon les revendications 1 à 6, par polymérisation d'un mélange comprenant.

A) au moins 90% en poids (par rapport au poids total de A et B) de méthacrylate de méthyle,

B) de 0 à 10% en poids (par rapport au poids total du mélange) d'un comonomère polymérisable avec le composé ci-dessus,

C) de 0,01 à 1% en poids (par rapport au poids total du mélange) d'une amine à empêchement stérique, ayant la formule donnée dans la revendication 1,

D) un amorceur radicalaire ou un système amorceur radicalaire courant, caractérisé en ce qu'on ajoute au mélange de 1 à 10% en poids (par rapport ou poids total) d'un plastifiant aliphatique ayant un point d'ébullition supérieur à 120°C et compatible avec le poly(méthacrylate de méthyle).

9. Procédé selon la revendication 8, caractérisé en ce que la polymérisation est mise en œuvre dans une chambre plate, formée de deux plaques de verre parallèles, disposées à une distance réciproque de 2 à 20 mm, et d'un profile pour étanchéité, courant sur le bord.

10. Solarium contenant une plaque de matière plastique selon la revendication 7.